# FASCICULE DE BREVET EUROPEEN

(11) **EP 3 625 779 B1**
(45) Date de publication et mention de la délivrance du brevet: **07.07.2021**
(21) Numéro de dépôt: 18720329.4
(22) Date de dépôt: 13.04.2018
(51) Int. Cl.: G08B 1/08, A61F 11/04, G02C 11/06, G09B 21/00, H04R 25/00

(54) **PROCÉDÉ ET ÉQUIPEMENT D'ASSISTANCE AUDITIVE**
HÖRHILFEVERFAHREN UND -VORRICHTUNG
HEARING ASSISTANCE METHOD AND APPARATUS

(30) Priorité: 16.05.2017 FR 1754292
(43) Date de publication de la demande: 25.03.2020
(73) Titulaire: ORANGE, 75015 Paris (FR)
(72) Inventeur: GAILLET, Thierry, 92326 Châtillon Cedex (FR)
(86) Numéro de dépôt international: PCT/FR2018/050935
(87) Numéro de publication internationale: WO 2018/211187

(56) Documents cités:
- DE-A1-102008 030 404
- ES-A1- 2 347 517
- US-A- 5 029 216
- US-A- 5 651 070
- US-A1- 2017 085 998
- US-A1- 2017 110 031

## Description

La présente invention concerne le domaine de la signalisation lumineuse, notamment pour informer visuellement un utilisateur de la position d'une source d'un signal sonore.

Pour des individus dont les capacités auditives sont diminuées, il est avantageux de fournir des informations pertinentes et facilement interprétables sur les sons environnants.

La diminution des capacités auditives d'un individu peut être due à des causes de natures très différentes. Par exemple, elle peut correspondre à un état pathologique de l'individu, comme une surdité. Elle peut aussi être liée à un contexte particulier : par exemple, un ouvrier portant un casque anti-bruit sur un chantier, un sujet écoutant de la musique par des écouteurs dans la rue, ou un sportif portant un casque de protection.

Les individus souffrant de surdité unilatérale ou de surdité bilatérale asymétrique importante (écart entre les seuils auditifs des deux oreilles supérieur à 20 dB), par exemple, sont confrontés à différents problèmes, comme une absence de localisation spatiale, une difficulté de compréhension d'une conversation en environnement bruyant, ou une absence de l'audition stéréophonique.

Chez ces individus, le cerveau perçoit des différences interaurales de temps et d'intensité qui ne sont pas dues aux situations sonores, mais à la pathologie auditive. Ainsi, quelle que soit la direction du son, ces individus localisent systématiquement le son du côté de la « meilleure » oreille, c'est-à-dire l'oreille non atteinte ou celle associée à la perte d'audition la plus faible.

Ainsi, il est intéressant, pour ces individus, d'être informés d'une position d'une source sonore, afin par exemple de pouvoir regarder dans la bonne direction.

Par ailleurs, ces individus, ainsi que les personnes portant un casque ou des écouteurs, peuvent être en danger dans de nombreuses situations de la vie quotidienne, par exemple lorsque des véhicules ou des objets arrivent hors ou à la limite de leur champ de vision.

Dans ces situations, le fait de ne pas entendre un son associé au danger (voiture qui freine brusquement, avertisseur sonore d'un véhicule, bruit d'un objet qui tombe sur un chantier ou interpellation d'un autre individu pour avertir d'un danger imminent) ou de ne pas pouvoir identifier rapidement sa provenance empêche ces personnes de réagir à temps pour éviter le danger.

Il est donc nécessaire de pouvoir informer ces personnes d'un son et/ou de la provenance d'un son.

Pour les personnes atteintes de surdité unilatérale ou bilatérale asymétrique importante, plusieurs solutions sont actuellement proposées. Certaines de ces solutions consistent en une transmission à la « meilleure » oreille des sons qui parviennent à l'oreille déficiente, par conduction osseuse, ou encore par transmission filaire ou sans fil (aide auditive CROS, pour « Contralateral Routing of Signal » en anglais, ou transmission controlatérale du signal en français).

Si la solution par transmission filaire ou sans fil est moins invasive que la solution par conduction osseuse, elle reste contraignante pour la personne qui porte le dispositif.

De plus, de telles solutions permettent d'augmenter la capacité auditive globale de la personne, mais ne résout pas le problème de la localisation spatiale et de l'identification de la provenance du son.

Enfin, ces solutions ne sont pas adaptées dans le cas d'une personne non atteinte de surdité, mais ayant une capacité auditive réduite temporairement due au port d'un casque, d'une protection auditive ou d'écouteurs.

Il y a ainsi un besoin pour avertir de manière simple et efficace un individu d'un son et/ou de sa provenance géographique.

ES 2 347 517 A1 divulgue un procédé d'assistance auditive, mis en œuvre par un dispositif informatique, le procédé comprenant :- réception de données relatives à un signal sonore ;- détermination d'une catégorie du signal sonore sur la base des données reçues ;- détermination d'une information représentative d'une position d'une source du signal sonore ;- détermination d'une consigne lumineuse en fonction de la catégorie du signal sonore déterminée et en fonction de l'information représentative de la position de la source ; et - transmission de la consigne lumineuse à au moins un émetteur lumineux.

US 2017/0110031 A1, US 5 651 070 A et DE 10 2008 030404 A1 divulguent également des dispositifs d'assistance basée sur l'analyse de signaux sonores.

La présente invention vient améliorer la situation.

Selon l'invention, il est proposé un procédé d'assistance auditive, mis en œuvre par un dispositif informatique, comprenant :
- réception de données relatives à un signal sonore ;
- réception (204) de données contextuelles associées aux données relatives au signal sonore reçues ;- détermination d'une catégorie du signal sonore sur la base des données reçues et des données contextuelles reçues;
- détermination d'une information représentative d'une position d'une source du signal sonore ;
- détermination d'une consigne lumineuse en fonction de la catégorie du signal sonore déterminée et en fonction de l'information représentative de la position de la source ; et
- transmission de la consigne lumineuse à au moins un émetteur lumineux.

Par « information représentative d'une position d'une source », on entend une information de position, absolue ou relative par rapport à l'utilisateur, sur la source émettant le signal sonore. Cette information peut être simplement une information du type « à gauche/à droite » de l'utilisateur, ou intégrer des données complémentaires, comme la distance estimée de la source, l'angle d'arrivée du signal sonore, etc.

Grâce à ce procédé, l'utilisateur est averti visuellement d'un son, et dispose d'une information simple pour localiser d'où provient le son.

Par « catégorie » on entend une modalité d'une variable qualitative associée à un ou plusieurs paramètres du signal sonore (intensité, fréquence, etc.), ou encore une classe de sons ou d'événements sonores (voix parlée, véhicule en marche, avertisseur sonore de voiture, etc.).

La détermination d'une catégorie de signal sonore permet d'envoyer une information pertinente à l'utilisateur.

L'utilisation de données contextuelles permet avantageusement d'adapter les catégories utilisées selon l'environnement de l'utilisateur.

Par exemple, si le procédé est mis en œuvre par un terminal mobile, il est possible, grâce au microphone du terminal mobile, de déterminer un niveau sonore moyen environnant, et de redéfinir les bornes des catégories associées au niveau sonore.

En effet, selon la catégorie déterminée, il peut être décidé d'émettre une consigne lumineuse pour l'allumage d'un émetteur lumineux, ou au contraire de ne pas émettre de consigne lumineuse (si le son correspond par exemple à un bruit ambiant).

Par ailleurs, la consigne lumineuse peut contenir une information de type « allumé/éteint », mais aussi des informations sur des paramètres du signal lumineux, comme une intensité ou un clignotement. Ainsi, ces paramètres peuvent aussi être adaptés en fonction de la catégorie déterminée.

Le dispositif informatique mettant en œuvre le procédé peut être, en tout ou partie, mis en œuvre à l'intérieur d'un objet appartenant à la catégorie des objets portables (ou « wearables » en anglais), tel que des lunettes, un couvre-chef ou un vêtement, ou intégré à un tel objet.

De manière alternative, le dispositif informatique mettant en œuvre le procédé est situé, en tout ou partie, dans un ordinateur type terminal mobile connecté à un objet portable, l'objet portable étant apte à transmettre des données relatives au signal sonore, ainsi qu'à recevoir une consigne lumineuse.

Dans un ou plusieurs modes de réalisation, la mise en œuvre du procédé proposé peut être répartie entre un processeur situé dans un objet portable ou intégré à l'objet portable, et un processeur d'un terminal mobile connecté à l'objet portable.

Selon un mode de réalisation, la détermination de la catégorie du signal sonore peut comprendre une sélection, parmi un ensemble de catégories prédéterminées, en fonction d'au moins une caractéristique du signal sonore.

Cette caractéristique du signal sonore peut comprendre l'un au moins parmi une durée, une fréquence, une intensité, un niveau d'énergie du signal sonore, un angle d'arrivée du signal sonore ou une quantité relative à une pression acoustique.

Cela permet une détermination de catégories qui peuvent être utilisées pour adapter au mieux le procédé proposé aux besoins d'un utilisateur.

Par exemple, différentes catégories (ou classes) relatives à un niveau sonore (en décibels, dB) peuvent être définies, et il peut être décidé de ne pas émettre un signal lumineux si le niveau est inférieur à 50 dB, d'émettre un signal lumineux avec une intensité modérée pour un niveau entre 50 dB et 90 dB, et un signal lumineux avec une intensité forte pour un niveau supérieur à 90 dB.

De manière alternative ou en complément, la détermination de la catégorie du signal sonore peut comprendre une recherche d'une signature sonore dans une base de données.

Par « signature », on entend un ensemble de paramètres permettant de caractériser une classe de sons.

Selon le mode de réalisation choisi, une signature peut par exemple comprendre une modélisation statistique de paramètres (ou indices acoustiques) caractéristiques d'une même classe de sons.

Ainsi, les catégories peuvent avantageusement être définies de manière plus précise, et venir éventuellement compléter ou être combinées avec l'utilisation des caractéristiques sonores telle qu'évoquée précédemment.

Par exemple, sur un chantier, où les niveaux sonores peuvent être globalement très élevés, il est possible de discriminer le son d'un objet lourd qui tombe au sol par rapport au son d'un marteau-piqueur.

Dans un ou plusieurs modes de réalisation, les données contextuelles peuvent comprendre des données de géolocalisation.

Par ailleurs, la consigne peut comprendre au moins un paramètre de commande de l'émetteur lumineux, le paramètre comprenant l'un au moins parmi une intensité lumineuse, une couleur ou une fréquence de clignotement.

Le paramètre de commande pourra ainsi être ajusté de manière pertinente en fonction de la catégorie de signal, ou encore à partir des données contextuelles. L'utilisateur dispose ainsi, de manière très rapide, d'une information beaucoup plus complète.

En outre, dans un ou plusieurs modes de réalisation, le procédé proposé pourra comprendre, suite à la transmission de la consigne lumineuse :
- une réception d'informations relatives à un mouvement d'un utilisateur suite à l'émission d'un signal lumineux par l'émetteur lumineux ;
- une détermination d'une consigne lumineuse mise à jour en fonction desdites informations relatives au mouvement de l'utilisateur ; et
- une transmission de la consigne lumineuse mise à jour à l'émetteur lumineux.

Cela permet de faire une boucle de rétrocontrôle et d'adapter la consigne lumineuse à la réaction de l'utilisateur (par exemple, extinction de l'émetteur lumineux si l'utilisateur a tourné la tête dans la direction indiquée, ou augmentation de l'intensité lumineuse si l'utilisateur n'a pas tourné la tête).

Selon un mode de réalisation, le procédé peut en outre comprendre une obtention d'informations relatives à un utilisateur et une réalisation d'une analyse prédictive sur lesdites informations récupérées. La consigne lumineuse est alors déterminée en outre en fonction de l'analyse prédictive réalisée.

L'analyse prédictive peut notamment être effectuée à partir d'algorithmes d'apprentissage statistique.

Ainsi, le système peut « apprendre » en fonction des réponses de l'utilisateur (par exemple en termes de délai mis pour tourner la tête après émission d'un signal lumineux, en fonction de son intensité, de l'angle d'arrivée du son, etc.), ainsi que de ses habitudes (type d'environnement sonore en fonction du jour de la semaine et/ou de l'heure de la journée et/ou de la localisation) pour adapter au mieux la consigne lumineuse à émettre.

Un avantage de ces algorithmes est d'adapter les analyses et les réponses, à partir des données reçues. Ils permettent de gérer un grand nombre de données (issues par exemple de différents capteurs du terminal mobile) et de réponses possibles de l'utilisateur.

Un autre avantage est l'aspect adaptatif des algorithmes d'apprentissage statistique : ces algorithmes sont capables de prendre en compte une évolution des informations d'entrée et de modifier en conséquence les réponses en sortie. Ainsi, le système d'analyse ou de réponse se retrouve amélioré, sans qu'aucune intervention d'un utilisateur ne soit nécessaire pour mettre à jour les données.

En fonction du mode de réalisation, les algorithmes d'analyse prédictive peuvent être réalisés par un processeur intégré au terminal mobile, ou par des serveurs distants (technique d'informatique en nuage, ou de « cloud computing » en anglais).

Dans un ou plusieurs modes de réalisation, le procédé peut en outre comprendre, suite à la transmission de la consigne lumineuse :
- une réception, via une interface homme machine, d'une donnée d'instruction d'un utilisateur suite à l'émission d'un signal lumineux par l'émetteur lumineux ;
- une détermination d'une consigne mise à jour en fonction de ladite donnée d'instruction reçue ; et
- une transmission de la consigne mise à jour à l'émetteur lumineux.

Par exemple, cette donnée d'instruction peut correspondre à une extinction du voyant lumineux.

Cette donnée d'instruction peut aussi être relative à un paramètre du signal lumineux (intensité maximale par exemple).

Selon un autre aspect, il est proposé un module d'un dispositif informatique pour assistance auditive, le module comprenant un circuit configuré pour :
- recevoir des données relatives à un signal sonore ;
- déterminer une catégorie du signal sonore sur la base des données reçues;
- déterminer une information représentative d'une position d'une source du signal sonore ;
- déterminer une consigne lumineuse en fonction de la catégorie du signal sonore déterminée et en fonction de l'information représentative de la position de la source ; et
- transmettre la consigne lumineuse à un émetteur lumineux.

Ce module peut être intégré à un dispositif, de préférence portable.

Ainsi, un autre aspect vise un dispositif informatique comprenant un module d'assistance auditive tel que proposé.

Selon un autre aspect, il est proposé un système comprenant :
- un dispositif informatique comprenant un module d'assistance auditive tel que proposé ; et
- un dispositif portatif connecté au dispositif informatique.

De préférence, le dispositif portatif comportera au moins un microphone et au moins un émetteur lumineux. Les données relatives au signal sonore pourront être obtenues à partir de signaux sonores respectivement acquis par l'au moins un microphone, et le dispositif informatique pourra être configuré pour transmettre la consigne lumineuse à l'au moins un émetteur lumineux dudit dispositif portatif.

Le dispositif portatif pourra être intégré à un objet portable, comme des lunettes ou un couvre-chef.

Un programme informatique, mettant en œuvre tout ou partie du procédé décrit ci-avant, installé sur un équipement préexistant, est en lui-même avantageux, dès lors qu'il permet une assistance auditive.

Ainsi, selon un autre aspect, il est proposé un programme d'ordinateur, chargeable dans une mémoire associée à un processeur, et comprenant des portions de code pour la mise en œuvre du procédé proposé lors de l'exécution dudit programme par le processeur, ainsi qu'un ensemble de données représentant, par exemple par voie de compression ou d'encodage, ledit programme d'ordinateur.

Ce programme pourra utiliser n'importe quel langage de programmation (par exemple, un langage objet ou autre), et être sous la forme d'un code source interprétable, d'un code partiellement compilé ou d'un code totalement compilé.

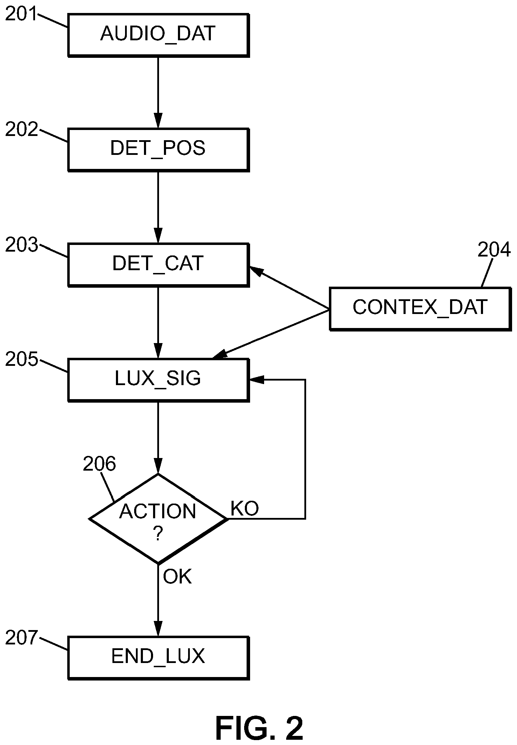

La figure 2, décrite en détails ci-après, peut former l'organigramme de l'algorithme général d'un tel programme informatique.

Un autre aspect concerne un support de stockage non-transitoire d'un programme exécutable par ordinateur, comprenant un ensemble de données représentant un ou plusieurs programmes, lesdits un ou plusieurs programmes comprenant des instructions pour, lors de l'exécution desdits un ou plusieurs programmes par un ordinateur comprenant une unité de traitement couplée de manière opérationnelle à des moyens mémoire et à un module d'interface entrées/sorties, pour exécuter tout ou partie du procédé décrit ci-avant.

D'autres caractéristiques et avantages de l'invention apparaîtront encore à la lecture de la description qui va suivre. Celle-ci est purement illustrative et doit être lue en regard des dessins annexés sur lesquels :
- la figure la illustre un équipement pour mettre en œuvre tout ou partie d'un procédé d'assistance auditive selon un mode de réalisation;
- la figure 1b illustre l'équipement représenté à la figure la, selon un autre angle de vue ;
- la figure 2 illustre un ordinogramme général dans un mode de réalisation particulier ;
- la figure 3 illustre un dispositif d'assistance auditive dans un mode de réalisation particulier.

La figure la illustre un équipement pour mettre en œuvre tout ou partie d'un procédé d'assistance auditive selon un mode de réalisation.

Sur la figure la, l'équipement 101 est représenté sous la forme d'une paire de lunettes. Cette représentation est donnée à titre d'exemple et de manière non limitative. L'équipement 101 peut se présenter sous la forme d'un autre objet appartenant à la technologie des « objets portables », ou « wearables » en anglais, comme :
- un couvre-chef (casque, chapeau, etc.) ;
- un vêtement (par exemple, une veste ou une chemise équipée d'un émetteur lumineux sur chaque manche) ;
- etc.

L'équipement 101 est muni d'au moins un microphone, et de préférence deux microphones.

Sur l'exemple de la figure la, l'équipement 101 est muni de deux microphones 102, 103. Il peut éventuellement y avoir trois ou plus de trois microphones. Par exemple, sur l'exemple de la figure la, il est possible d'ajouter un troisième microphone sur une partie «centrale» de la monture, comme par exemple entre les deux verres de lunettes.

Préférentiellement, deux des microphones sont situés de part et d'autre de l'équipement, de manière à respecter une configuration « gauche/droite » par rapport à l'utilisateur.

Par exemple, dans le cas d'un équipement se présentant sous la forme d'une paire de lunettes, deux microphones peuvent être présents de part de d'autre de la monture, comme par exemple sur les branches (un microphone sur chaque branche).

Dans le cas d'un couvre-chef, un microphone peut être placé à proximité de chacune des deux oreilles. Dans le cas d'un vêtement du type chemise ou veste, un microphone peut être placé, par exemple, au niveau de chaque épaule.

Les microphones 102, 103 sont aptes à recevoir des signaux sonores émis par une source sonore 105. Grâce à ces microphones, il est possible de localiser la source sonore 105. Par « localiser », on entend déterminer une information associée à la position spatiale de la source sonore. Une telle localisation peut être effectuée, par exemple, à partir de certains paramètres, tels que l'intensité de l'onde sonore à chaque microphone ou le temps d'arrivée de l'onde sonore à chaque microphone.

L'information associée à la position spatiale de la source peut être, de manière simple, de quel côté (au sens de « à droite » ou « à gauche ») se trouve la source par rapport à la personne qui porte l'équipement 101. De manière non limitative, ce «côté» peut être déterminé en comparant les paramètres précédemment évoqués.

Par exemple, si l'onde sonore parvient au microphone situé sur la gauche de l'équipement 101 (par exemple, sur la branche gauche des lunettes) avant de parvenir au microphone droit (situé sur la droite de l'équipement 101), il peut être décidé que la source 105 est située à gauche de l'utilisateur.

Selon un autre mode de réalisation, il peut être décidé que le côté où se trouve la source 105 correspond au côté du microphone recevant le signal sonore de plus forte intensité (l'intensité d'un son étant inversement proportionnelle au carré de la distance entre la source et le récepteur).

Selon un autre mode de réalisation, un seul microphone peut être utilisé en vue de déterminer une information associée à la position spatiale de la source sonore. Un tel microphone peut être de type ambisonique par exemple.

L'information associée à la position spatiale de la source peut alternativement être, de manière plus complète, la direction d'arrivée de l'onde sonore (azimut en coordonnées sphériques), ainsi qu'éventuellement la distance de la source. Ces quantités peuvent être évaluées par différentes méthodes, notamment des méthodes basées sur l'ILD (pour « Interaural Level Différence » en anglais, ou « différence de niveau interaurale » en français), et l'ITD (pour « Interaural Time Différence » en anglais, ou « différence de temps interaurale » en français).

De manière plus complète, des indices spectraux caractérisés par des fonctions de transfert relatives à la tête (ou HRTF pour « Head-Related Transfer Function » en anglais) peuvent aussi être utilisés pour améliorer la localisation (notamment en affinant la position verticale, ou en supprimant les confusions dites « avant-arrière »).

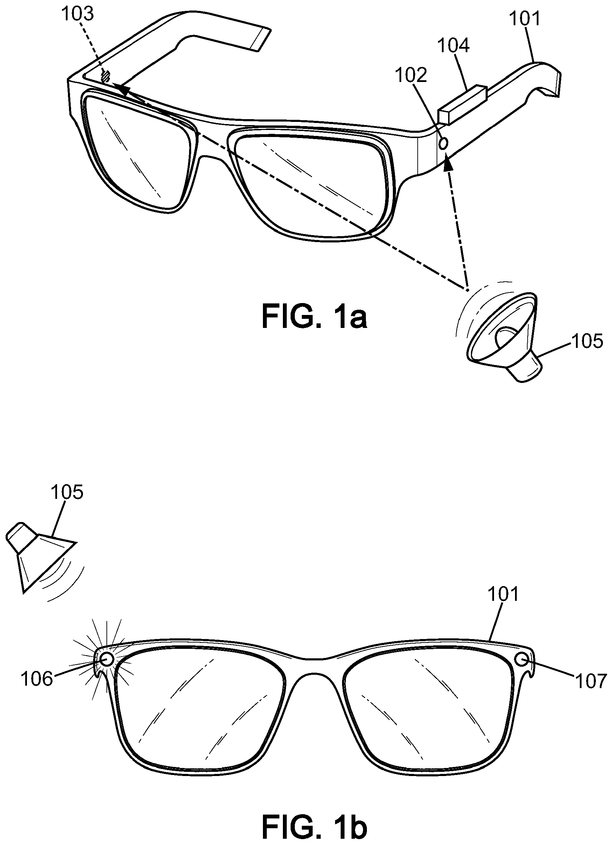

La figure 1b illustre l'équipement représenté à la figure la, selon un autre angle de vue. L'équipement est représenté de l'intérieur, par rapport à un utilisateur. L'élément 106 est donc sur la gauche, à l'intérieur de la monture, et l'élément 107 est sur la droite, à l'intérieur de la monture.

L'équipement 101 est muni d'au moins un émetteur lumineux.

Dans le mode de réalisation préférentiel de la figure 1b, l'équipement est muni de deux émetteurs lumineux 106, 107.

Ces émetteurs lumineux peuvent être par exemple des diodes électroluminescentes (ou LED, pour « Light-Emitting Diode » en anglais).

Deux des émetteurs lumineux sont situés préférentiellement de part et d'autre de l'équipement 101 par rapport à un axe vertical. Ainsi, un émetteur lumineux 106 se situe sur un côté gauche de l'équipement 101, et un émetteur lumineux 107 se situe sur un côté droit de l'équipement 101.

Sur la figure 1b, les émetteurs lumineux 106 et 107 sont situés à l'intérieur de l'équipement 101 par rapport à la tête de l'utilisateur. Alternativement, ces émetteurs lumineux peuvent être disposés à un endroit quelconque, mais toujours de manière à ce que l'utilisateur qui porte l'équipement puisse voir un signal lumineux émis par l'un des émetteurs.

Lorsque l'équipement est un couvre-chef (casque ou casquette par exemple), les émetteurs lumineux peuvent être situés par exemple de chaque côté de la visière, sur le côté inférieur de la visière (« en-dessous »), de manière à ce que l'utilisateur puisse les voir. Lorsque l'équipement est un vêtement type chemise ou veste, les émetteurs lumineux peuvent être situés sur les manches.

Selon un mode de réalisation préférentiel, lorsqu'il est déterminé (à partir des signaux reçus par les microphones, par une méthode de localisation de la source telle que présentée avant) qu'un son arrive en provenance d'une direction donnée (droite/gauche), l'émetteur lumineux situé dans cette même direction émet un signal lumineux. Par exemple, si une source sonore 105 est située à gauche de l'utilisateur, comme représenté à la figure 1b, la LED 106 située sur la gauche de la monture s'allume.

Alternativement, l'équipement 101 possède un seul émetteur lumineux. Cet unique émetteur peut être situé, par exemple, du côté de l'oreille faible pour une personne atteinte de surdité unilatérale ou asymétrique. Cet émetteur peut aussi être situé sur un endroit quelconque de l'équipement 101, et indiquer la position de la source sonore, par exemple, par une couleur (vert pour un signal sur la droite, rouge pour un signal sur la gauche).

L'équipement 101 permet donc une assistance auditive via l'émission de signaux lumineux. En effet, avec un tel équipement, les personnes sont informées qu'un signal sonore a été émis, ainsi que de sa provenance.

Ainsi, les personnes atteintes de surdité unilatérale ou bilatérale asymétrique, qui entendent le son mais ne parviennent pas à le localiser, sont informées de manière simple et efficace de la provenance du son.

Les personnes ayant un casque anti-bruit, des écouteurs, un casque de moto, ou tout équipement qui diminue leurs capacités auditives, peuvent être averties d'un son et savoir rapidement d'où le son provient. Cela peut être très utile lorsque la personne est appelée par une autre, ou encore quand un danger approche (objet qui tombe sur un chantier, voiture qui freine brusquement, etc.).

Selon une réalisation possible, l'émetteur lumineux n'est allumé que lorsque la source a été identifiée comme « suffisamment » à gauche ou à droite de l'utilisateur. Par « suffisamment », on entend que l'angle d'arrivée est tel qu'il est difficile pour l'utilisateur de percevoir visuellement la source (source en dehors du champ de vision ou près de la limite du champ de vision). Cela permet, par exemple, qu'aucun allumage d'émetteur lumineux ne soit effectué lorsqu'un utilisateur atteint de surdité est en conversation avec des personnes situées en face de lui, qui parlent tour à tour.

L'angle d'arrivée du son par rapport à la tête (modélisée par exemple par un point d'une monture de lunettes au-dessus du nez, ou par un point situé sur un axe vertical médian de la visière d'un casque ou d'une casquette, etc.) au-delà duquel une consigne lumineuse doit être émise peut être préréglée, et/ou modifiée par l'utilisateur selon ses préférences d'utilisation.

Il peut aussi être décidé de n'émettre une consigne lumineuse que lorsque le signal sonore reçu a une intensité sonore supérieure à une valeur prédéterminée ou fixée par l'utilisateur. Ainsi, si le son n'est pas assez « fort », aucun signal lumineux ne sera émis.

Dans un mode de réalisation alternatif, plusieurs émetteurs lumineux peuvent s'allumer simultanément, par exemple lorsqu'un son a été détecté à l'arrière de l'individu, ou encore lorsque deux sons proviennent de deux directions différentes (l'un de la droite, l'autre de la gauche). Dans cette dernière situation, il est possible d'émettre deux signaux lumineux avec des intensités différentes. Par exemple, une intensité plus élevée peut correspondre à une source située à plus faible distance.

En référence à nouveau à la figure la, l'équipement 101 comporte un dispositif de commande 104 intégrant un microprocesseur apte à mettre en œuvre tout ou partie du procédé proposé, dont un exemple de mise en œuvre est décrit de manière détaillée en référence à la figure 2.

De manière non limitative, le dispositif de commande 104 peut se présenter sous la forme d'un petit boitier fixé sur l'équipement 101, comme illustré sur la figure la, ou être intégré directement à l'équipement 101 de manière à ne pas être visible (par exemple, à l'intérieur d'une branche de paire de lunettes).

Le dispositif de commande 104 peut comprendre un processeur couplé de manière opérationnelle à une mémoire, qui peuvent, dans un ou plusieurs modes de réalisation, être intégrés au sein d'un microcontrôleur du dispositif de commande 104. Selon le mode de réalisation choisi, le dispositif de commande 104 peut comprendre une interface d'entrée pour recevoir des données associées à un ou plusieurs signaux sonores, ainsi qu'une interface de sortie pour envoyer une commande relative à un ou plusieurs signaux lumineux.

Selon un mode de réalisation, le dispositif de commande 104 peut en outre intégrer une interface de communication sans fil pour communiquer avec un terminal mobile tel qu'un « téléphone intelligent » (ou « smartphone » en anglais), ou avec un serveur distant. Le protocole de communication sans fil peut utiliser un réseau longue portée, comme LoRa ou Sigfox. Il peut aussi, de manière préférentielle, utiliser un réseau courte portée, comme le Wi-Fi, Z-Wave, ZigBee, Bluetooth, ou encore le BLE (pour « Bluetooth Low Energy » en anglais, ou Bluetooth basse énergie en français) qui permet un débit de communications de données du même ordre de grandeur que le Bluetooth pour une consommation d'énergie nettement inférieure.

Dans un mode de réalisation, la détermination d'une localisation associée au signal sonore peut être mise en œuvre par un microprocesseur ou un microcontrôleur du dispositif de commande 104. Alternativement, le dispositif de commande 104 envoie des données relatives au signal sonore à un terminal mobile auquel il est connecté, et la détermination de la localisation est effectuée par une unité de traitement (intégrant un ou plusieurs processeurs) du terminal mobile.

Selon un mode de réalisation, le dispositif de commande 104 (en référence à la figure la) est configuré pour envoyer une consigne lumineuse pour l'éclairage d'un émetteur lumineux 106, 107. Alternativement, le terminal mobile connecté au dispositif de commande 104 est configuré pour envoyer la consigne, par exemple par le biais d'une application logicielle exécutée sur le terminal mobile.

Dans un mode de réalisation particulier, le dispositif de commande 104 comprend une interface homme-machine, par exemple un ou plusieurs boutons que l'utilisateur peut actionner. Ainsi, un individu peut, par exemple, arrêter l'émission du signal lumineux (c'est-à-dire éteindre la LED, par exemple s'il a bien pris connaissance de la localisation du signal sonore), ou régler certains paramètres, comme l'intensité maximale d'un signal lumineux. De manière alternative ou en complément, ces consignes de fonctionnement peuvent être envoyées à un terminal mobile connecté au dispositif de commande 104 via une interface homme-machine du terminal (par exemple, un écran tactile) - grâce à une application dédiée notamment aux réglages des paramètres ou au contrôle des signaux lumineux.

La figure 2 illustre un ordinogramme du procédé proposé dans un ou plusieurs modes de réalisation.

En référence à la figure la, le procédé illustré sur la figure 2 peut être mis en œuvre, en tout ou partie, soit par un processeur ou un microcontrôleur du dispositif de commande 104, soit par un processeur d'un terminal mobile connecté au dispositif de commande 104. Alternativement, la mise en œuvre du procédé proposé peut être répartie entre le dispositif de commande 104 et le terminal mobile.

Le terminal mobile reçoit (201), en provenance du dispositif de commande 104, des données relatives à un ou plusieurs signaux sonores captés par les microphones 102, 103. Ces données permettent notamment de déterminer une information sur la position des sources respectives des signaux sonores (202), comme décrit précédemment.

Ces données permettent aussi de déterminer une catégorie de chaque signal sonore reçu (203).

Cela permet avantageusement :
- d'éviter que les émetteurs lumineux soient allumés de manière intempestive, ce qui nuirait au confort de l'utilisateur ; et
- d'adapter la consigne lumineuse à la catégorie du signal sonore, de manière à fournir une information pertinente et adaptée à l'utilisateur via l'émission de signal lumineux.

Selon un mode de réalisation pour la détermination d'une ou plusieurs catégories pour chaque signal sonore reçu (203), les catégories de signal sonore (ou catégories de son) peuvent être des classes représentant des plages de valeurs associées à des paramètres sonores. Ces paramètres sonores peuvent être, de manière non limitative, une durée, une fréquence, une intensité, un niveau d'énergie du signal sonore, un angle d'arrivée du signal sonore, ou une quantité relative à une pression acoustique.

Des catégories peuvent notamment être définies selon un niveau de bruit exprimé en décibels dB (mesure relative à la pression acoustique), par exemple : « faible » entre 0 dB et 20 dB, « modéré » entre 20 dB et 60 dB, « assez élevé » entre 60 dB et 90 dB, et « élevé » pour plus de 90 dB.

Il peut notamment être décidé de ne pas émettre de signal lumineux si le niveau sonore appartient aux catégories « faible » et « modéré », d'émettre un signal lumineux d'intensité légère pour un niveau « assez élevé », et d'émettre un signal lumineux de forte intensité pour un niveau « élevé ».

Bien entendu, d'autres catégories peuvent être définies, en utilisant des valeurs limites différentes. Par ailleurs, des catégories peuvent être définies à partir d'autres paramètres parmi les paramètres précités.

Notamment, comme évoqué précédemment, il est possible de définir des catégories correspondant à des angles d'arrivée du signal sonore, dont une catégorie « en dehors ou proche de la limite du champ de vision », et de décider de n'émettre un signal lumineux que si le signal sonore reçu se situe dans cette catégorie.

Il est possible d'utiliser plusieurs catégories relatives chacune à un paramètre du signal sonore pour le signal lumineux émis. Par exemple, un signal sonore de niveau « assez élevé » dont la durée est supérieure à un seuil prédéterminé peut être associé à un signal lumineux d'intensité légère avec un clignotement - pour attirer l'attention de la personne qui porte l'équipement - etc.

Un ensemble de catégories de signal sonore, associées à un ou plusieurs paramètres acoustiques, peut être prédéterminé, et la détermination d'une ou plusieurs catégories pour chaque signal sonore reçu (203) peut comprendre une sélection dans cet ensemble d'au moins une catégorie. Bien entendu, une ou plusieurs catégories peuvent être considérées pour le traitement de chaque signal sonore reçu (par exemple, une catégorie pour le volume sonore, et une catégorie pour l'angle d'arrivée).

Selon un autre mode de réalisation pour la détermination d'une ou plusieurs catégories pour chaque signal sonore reçu (203), alternatif ou complémentaire au premier mode de réalisation, le signal sonore est catégorisé en « bruit ambiant » / « n'appartenant pas au bruit ambiant ».

Cette catégorisation peut être effectuée par une analyse par le processeur, sur une période de temps prédéterminée (de l'ordre par exemple de quelques secondes), du contexte sonore. Il peut être décidé par exemple que le signal sonore correspond à un « bruit ambiant » si son niveau sonore correspond au niveau sonore moyen de la séquence analysée.

Ainsi, si le signal sonore est identifié comme faisant partie du bruit ambiant, aucun signal lumineux n'est émis.

Selon un autre mode de réalisation pour la détermination d'une ou plusieurs catégories pour chaque signal sonore reçu (203), alternatif ou complémentaire aux modes de réalisation décrits ci-dessus, la catégorie (ou « classe ») de son correspond à un type précis de son, par exemple « conversation », « véhicule qui roule », « crissement de pneus », etc.

Selon ce mode de réalisation, la détermination 203 de la catégorie du signal sonore est effectuée grâce à une signature sonore, déterminée à partir d'une analyse spectrale du son, et notamment de sa pression acoustique par exemple.

Par « signature », on entend un ensemble de paramètres permettant de caractériser une classe de sons. Plus spécifiquement, une signature peut comprendre une modélisation statistique de paramètres (ou indices acoustiques) caractéristiques de l'enveloppe spectrale ou de la répartition de la pression acoustique de signaux sonores issus d'une même classe de sons. Cette modélisation peut être obtenue grâce à des algorithmes d'analyse prédictive (apprentissage statistique ou automatique).

En effet, le sonagramme (ou sonogramme), qui représente la pression acoustique d'une onde sonore (ou la tension associée) en fonction du temps, a une forme qui est spécifique à la classe de sons : voiture qui roule, bruit de freinage, voix humaine, etc.

Ainsi, chaque classe de sons possède une signature qui lui est propre.

Selon un mode de réalisation, un ensemble de signatures prédéfinies, associées à différentes classes (ou catégories) de sons, sont enregistrées dans une base de données.

Lorsque le système reçoit un signal sonore, une analyse (basée par exemple sur des algorithmes d'intelligence artificielle, d'apprentissage ou de classification) est effectuée sur ce signal pour en extraire un ensemble de paramètres, et déterminer le modèle statistique qui s'applique le mieux

(par exemple, un maximum de vraisemblance). Autrement dit, le signal sonore entrant est associé à une signature de la base de données, et donc à une classe de sons.

Des algorithmes d'apprentissage statistique peuvent être utilisés pour mettre à jour la base de données de signatures.

Une consigne lumineuse est envoyée (205) vers l'au moins un émetteur lumineux de l'équipement 101.

Cette consigne lumineuse est déterminée en fonction de la catégorie de signal sonore déterminée (203), ainsi que de l'information sur la position de la source déterminée (202).

Comme mentionné précédemment, une consigne lumineuse est envoyée pour commander un allumage d'au moins un émetteur lumineux de l'équipement 101, notamment en fonction de la position d'un son reçu. Selon un mode de réalisation très simple, un émetteur lumineux situé sur la gauche (resp. la droite) de l'équipement 101 émet un signal lumineux lorsque le signal sonore reçu a été localisé à gauche (resp. à droite) de l'utilisateur - lorsque ce signal sonore correspond à une catégorie pour laquelle il convient d'émettre un signal lumineux, comme décrit précédemment.

La catégorie de signal sonore permet de déterminer s'il convient d'émettre un signal lumineux (par exemple, il n'y a pas d'allumage d'une LED si le signal sonore correspond à du «bruit ambiant »).

Optionnellement, elle est utilisée pour déterminer des paramètres du signal lumineux émis (couleur, intensité, clignotement, durée d'allumage, etc.).

Par exemple, l'une des catégories peut correspondre à de la « conversation », c'est-à-dire à de la parole dans une bande d'intensité moyenne (une intensité plus élevée correspondrait plutôt à un cri). Dans ce cas, le signal lumineux émis peut être de faible intensité, et/ou associé à une couleur appropriée (du vert par exemple).

Selon un autre exemple, et de manière non limitative, une catégorie peut être « objet lourd qui tombe au sol », ou « freinage brutal », ou de manière plus générale un « danger ». Dans ce cas, le signal lumineux émis peut être de forte intensité, et/ou associé à un clignotement, et/ou d'une couleur appropriée (du rouge par exemple).

Optionnellement, des données contextuelles sont obtenues (204).

Ces données contextuelles peuvent être :
- une position géographique (par ex., données de géolocalisation, obtenues par exemple par un récepteur GPS (pour « Global Positioning System » en anglais, ou Système global de positionnement en français) ou par un récepteur du système de navigation par satellites Galileo) ;
- une donnée temporelle (par ex., heure de la journée) ;
- une donnée météorologique ;
- etc.

Les données contextuelles peuvent être utilisées dans la détermination de la catégorie de signal sonore.

Par exemple, les valeurs limites des classes des niveaux sonores (en dB) peuvent être définies en fonction de données de géolocalisation : dans une rue très animée d'une grande ville, le niveau sonore moyen est très supérieur au niveau sonore moyen dans un village en zone rurale.

Selon un autre exemple non limitatif, une base de données des signatures sonores peut aussi être définie à partir d'une position géographique, les sons « habituels » n'étant pas les mêmes selon les différents environnements.

Les données contextuelles peuvent aussi être utilisées dans la détermination de valeurs de paramètres de la consigne lumineuse.

Par exemple, si des données météorologiques indiquent un temps très ensoleillé, l'intensité lumineuse du signal peut être augmentée (pour pouvoir être repéré dans un environnement très lumineux). A l'inverse, en pleine nuit, l'intensité lumineuse peut être diminuée, pour ne pas aveugler l'utilisateur.

De manière optionnelle, suite à la transmission de la consigne lumineuse (205), il peut être vérifié si une action est effectuée (206).

Cette action peut être un mouvement d'un utilisateur.

Par exemple, en référence à la Fig. la, l'équipement 101 peut être muni d'un accéléromètre pour capter un mouvement de tête d'un utilisateur. Ainsi, le système peut déterminer si l'utilisateur tourne la tête du côté pour lequel un signal lumineux a été émis.

Si aucun mouvement de tête n'a été détecté (KO), la consigne lumineuse peut être renouvelée ou mise à jour. La mise à jour peut concerner certains paramètres. Par exemple, l'intensité lumineuse peut être augmentée, ou un clignotement peut être mis en œuvre.

Si un mouvement de tête a été détecté (OK), le système peut commander une extinction de la LED.

Dans un autre mode de réalisation, l'équipement 101 peut aussi être muni d'un détecteur de mouvements des yeux. Là encore, la consigne lumineuse peut être renouvelée, mise à jour, ou interrompue en fonction d'une détection du mouvement des yeux vers la position détectée de la source lumineuse.

Cette action peut, alternativement ou en complément, être une commande d'extinction du signal lumineux par l'utilisateur. Cette commande peut être envoyée par l'utilisateur via une interface homme-machine de l'équipement (par exemple, un bouton « marche/arrêt » sur le boîtier du dispositif 104 de la figure la) ou du terminal mobile (par exemple, un écran tactile).

Sur réception de cette commande (OK), le système peut commander une extinction de la LED (207).

Si une telle commande d'extinction n'est pas reçue, la consigne lumineuse peut être renouvelée.

Il peut aussi être décidé que si aucune action n'a été détectée pendant une certaine période (206), la consigne lumineuse est mise à jour (par exemple, augmentation de l'intensité).

Selon un mode de réalisation particulier, le procédé proposé peut être adapté à l'utilisateur grâce à des techniques d'analyse prédictive (apprentissage statistique par exemple).

Par exemple, le système peut « apprendre » certaines habitudes de l'utilisateur, comme son temps moyen de réaction à un signal lumineux, les niveaux sonores moyens auxquels l'utilisateur est confronté en fonction de la date et de l'heure, l'intensité lumineuse la mieux adaptée, l'angle d'arrivée limite moyen au-delà duquel l'utilisateur a besoin d'une assistance auditive, etc.

Ainsi, la consigne lumineuse émise, ainsi que les paramètres de commande associés (intensité, fréquence de clignotement, etc.) sont ajustés de manière automatique par le système de manière à être adaptés au mieux aux besoins de l'utilisateur.

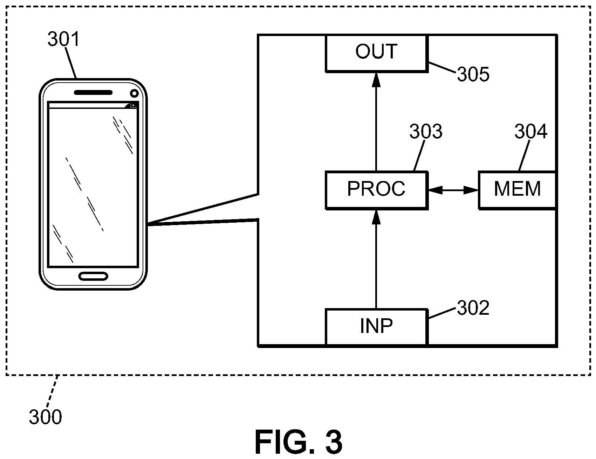

La figure 3 représente un exemple de dispositif d'assistance auditive, dans un mode de réalisation particulier.

Dans ce mode de réalisation, le dispositif 300 comprend une mémoire 304 pour stocker des instructions permettant la mise en œuvre du procédé, les informations reçues, et des données temporaires pour réaliser le procédé proposé tel que décrit précédemment. Le dispositif 300 comprend en outre un circuit de commande 303, une interface d'entrée 302 pour la réception de données de référence, et une interface de sortie 305 pour la fourniture d'une consigne lumineuse.

Le dispositif 300 peut se présenter, pour permettre une interaction aisée avec un utilisateur, sous la forme d'un terminal mobile muni d'un écran tactile 301. Bien entendu, le clavier est facultatif, notamment dans le cadre d'un ordinateur ayant la forme d'une tablette tactile, par exemple.

En fonction du mode de réalisation, le dispositif 300 peut être un terminal mobile, un ordinateur, un réseau d'ordinateurs, un composant électronique, ou un autre appareil comprenant un processeur couplé de manière opérationnelle à une mémoire, ainsi que, selon le mode de réalisation choisi, une unité de stockage de données, et d'autres éléments matériels associés comme une interface de réseau et un lecteur de support pour lire un support de stockage amovible et écrire sur un tel support (non représentés sur la figure). Le support de stockage amovible peut être, par exemple, un disque compact (CD), un disque vidéo/polyvalent numérique (DVD), un disque flash, une clé USB, etc. En fonction du mode de réalisation, la mémoire, l'unité de stockage de données ou le support de stockage amovible contient des instructions qui, lorsqu'elles sont exécutées par le circuit de commande 303, amènent ce circuit de commande 303 à effectuer ou contrôler les parties interface d'entrée 302, interface de sortie 305, stockage de données 304 et/ou traitement de données des exemples de mise en œuvre du procédé proposé décrits dans les présentes. Le circuit de commande 303 peut être un composant implémentant un processeur ou une unité de calcul pour une assistance auditive selon le procédé proposé et le contrôle des unités 302, 304 et 305 du dispositif 300.

En outre, le dispositif 300 peut être mis en œuvre sous forme logicielle (« software » ou « firmware »), auquel cas il prend la forme d'un programme exécutable par un processeur, correspondant par exemple à une application téléchargeable et exécutable sur un équipement de type smartphone ou tablette, comme décrit ci-dessus, ou sous forme matérielle (ou « hardware »), comme un circuit intégré spécifique application (ASIC), un système sur puce (SOC), ou sous forme d'une combinaison d'éléments matériels et logiciels, comme par exemple un programme logiciel destiné à être chargé et exécuté sur un composant de type FPGA (Field Programmable Gate Array). Les SOC (System On Chip) ou système sur puce sont des systèmes embarqués qui intègrent tous les composants d'un système électronique dans une puce unique. Un ASIC (Application Specific Integrated Circuit) est un circuit électronique spécialisé qui regroupe des fonctionnalités sur mesure pour une application donnée. Les ASIC sont généralement configurés lors de leur fabrication et ne peuvent être que simulés par l'utilisateur. Les circuits logiques programmables de type FPGA (Field-Programmable Gate Array) sont des circuits électroniques reconfigurables par l'utilisateur.

Le dispositif 300 peut également utiliser des architectures hybrides, comme par exemple des architectures basées sur un CPU+FPGA, un GPU (Graphics Processing Unit) ou un MPPA (Multi-Purpose Processor Array).

Par ailleurs, le schéma fonctionnel présenté sur la figure 2 est un exemple typique d'un programme dont certaines instructions peuvent être réalisées au sein de l'équipement décrit. A ce titre, la figure 2 peut correspondre à l'organigramme de l'algorithme général d'un programme informatique dans un mode de réalisation particulier.

Bien entendu, la présente invention ne se limite pas aux formes de réalisation décrites ci-avant à titre d'exemples ; elle s'étend à d'autres variantes.

D'autres réalisations sont possibles.

Par exemple, un dispositif portatif intégrant un module d'assistance selon un mode de réalisation peut comporter une interface de communication sans fil pour communiquer avec un serveur distant.

Ce mode de réalisation est particulièrement avantageux dans le cadre d'une gestion centralisée d'un ensemble d'équipements, utilisés par exemple par un ensemble de personnes sur un chantier ou dans une usine. Le serveur distant peut alors recevoir des données provenant des différents équipements, et les analyser pour en retirer des informations pertinentes.

Un tel mode de réalisation peut être utilisé dans le cadre d'une application de gestion de terminaux mobiles (ou « device mobile management » en anglais), qui permet notamment :
- de mettre à jour l'ensemble des applications installées sur les terminaux mobiles (dans le cas où l'équipement est relié à un terminal mobile) ;
- de contrôler à distance les équipements - par exemple, si une défaillance est observée à un endroit du chantier, un signal lumineux d'avertissement peut être envoyé à des équipements situés à d'autres endroits du chantier ;
- de vérifier que toutes les personnes ont réagi (par exemple d'un mouvement de tête) après l'émission d'un signal lumineux relatif à un bruit identifié comme provenant d'un « danger » ;
- de « croiser » (ou corréler) des informations reçues pour préciser une information - par exemple, il est possible, à distance, de localiser précisément une chute d'un objet lourd sur un chantier en utilisant conjointement les signaux sonores reçus par différents équipements et leur données de géolocalisation.

Un tel mode de réalisation permet aussi d'enrichir en temps réel des bases de données stockant par exemple des signatures sonores, des coordonnées de géolocalisation, ou toute autre information pouvant être utilisée dans le cadre d'un apprentissage statistique ou du « big data » (littéralement « grosses données » en français, ou mégadonnées).

Les informations envoyées par les différents équipements au serveur distant peuvent aussi être utilisées dans le cadre d'une analyse « post-événement », par exemple pour un audit après incident.

En fonction du mode de réalisation choisi, certains actes, actions, événements ou fonctions de chacune des méthodes décrites dans le présent document peuvent être effectués ou se produire selon un ordre différent de celui dans lequel ils ont été décrits, ou peuvent être ajoutés, fusionnés ou bien ne pas être effectués ou ne pas se produire, selon le cas. En outre, dans certains modes de réalisation, certains actes, actions ou évènements sont effectués ou se produisent concurremment et non pas successivement.

Bien que décrits à travers un certain nombre d'exemples de réalisation détaillés, le procédé proposé et l'équipement pour la mise en œuvre du procédé comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent. De plus, différents aspects et caractéristiques décrits ci-dessus peuvent être mis en œuvre ensemble, ou séparément, ou bien substitués les uns aux autres, et l'ensemble des différentes combinaisons et sous combinaisons des aspects et caractéristiques font partie de la portée de l'invention. En outre, il se peut que certains systèmes et équipements décrits ci-dessus n'incorporent pas la totalité des modules et fonctions décrits pour les modes de réalisation préférés.

## Revendications

1. Procédé d'assistance auditive, mis en œuvre par un dispositif informatique (300), le procédé comprenant :
- réception (201) de données relatives à un signal sonore ;
- réception (204) de données contextuelles associées aux données relatives au signal sonore reçues ;
- détermination (203) d'une catégorie du signal sonore sur la base des données reçues et des données contextuelles reçues;
- détermination (202) d'une information représentative d'une position d'une source (105) du signal sonore ;
- détermination (205) d'une consigne lumineuse en fonction de la catégorie du signal sonore déterminée et en fonction de l'information représentative de la position de la source (105) ; et
- transmission de la consigne lumineuse à au moins un émetteur lumineux (106, 107).

2. Procédé selon la revendication 1, dans lequel la détermination (203) de la catégorie du signal sonore comprend une sélection, parmi un ensemble de catégories prédéterminées, en fonction d'au moins une caractéristique du signal sonore.

3. Procédé selon la revendication 2, dans lequel l' au moins une caractéristique du signal sonore comprend l'un au moins parmi une durée, une fréquence, une intensité, un niveau d'énergie du signal sonore, un angle d'arrivée du signal sonore ou une quantité relative à une pression acoustique.

4. Procédé selon l'une des revendications précédentes, dans lequel la détermination (203) de la catégorie du signal sonore comprend une recherche d'une signature sonore dans une base de données.

5. Procédé selon l'une des revendications précédentes, dans lequel les données contextuelles comprennent des données de géolocalisation.

6. Procédé selon l'une des revendications précédentes, dans lequel la consigne comprend au moins un paramètre de commande de l'émetteur lumineux, le paramètre comprenant l'un au moins parmi une intensité lumineuse, une couleur ou une fréquence de clignotement.

7. Procédé selon l'une des revendications précédentes, comprenant en outre, suite à la transmission de la consigne lumineuse :
- réception (206) d'informations relatives à un mouvement d'un utilisateur suite à l'émission d'un signal lumineux par l'émetteur lumineux ;
- détermination (205) d'une consigne lumineuse mise à jour en fonction desdites informations relatives au mouvement de l'utilisateur ; et
- transmission de la consigne lumineuse mise à jour à l'émetteur lumineux (106, 107).

8. Procédé selon l'une des revendications précédentes, comprenant en outre :
- obtention d'informations relatives à un utilisateur ; et
- réalisation d'une analyse prédictive sur lesdites informations récupérées ;
dans lequel la consigne lumineuse est déterminée (205) en outre en fonction de l'analyse prédictive réalisée.

9. Procédé selon l'une des revendications précédentes, comprenant en outre, suite à la transmission de la consigne lumineuse :
- réception (206), via une interface homme machine, d'une donnée d'instruction d'un utilisateur suite à l'émission d'un signal lumineux par l'émetteur lumineux ;
- détermination (205) d'une consigne mise à jour en fonction de ladite donnée d'instruction reçue ; et
- transmission de la consigne mise à jour à l'émetteur lumineux (106, 107).

10. Module d'un dispositif informatique (300) pour assistance auditive, le module comprenant un circuit configuré pour la mise en œuvre d'un procédé selon l'une quelconque des revendications 1 à 9.

11. Dispositif informatique (300) comprenant un module d'assistance auditive selon la revendication 10.

12. Système comprenant :
- un dispositif informatique (300) selon la revendication 11 ; et
- un dispositif portatif (101) connecté au dispositif informatique, dans lequel le dispositif portatif comporte au moins un microphone (102, 103) et au moins un émetteur lumineux (106, 107), les données relatives au signal sonore étant obtenues à partir du signal sonore acquis par l'au moins un microphone (102, 103) ;
dans lequel le dispositif informatique (300) est configuré pour transmettre la consigne lumineuse à l'au moins un émetteur lumineux (106, 107) dudit dispositif portatif (101).

13. Programme d'ordinateur, chargeable dans une mémoire associée à un processeur, et comprenant des portions de code pour la mise en œuvre d'un procédé selon l'une quelconque des revendications 1 à 9 lors de l'exécution dudit programme par le processeur.

14. Support de stockage non-transitoire d'un programme exécutable par ordinateur, comprenant un ensemble de données représentant un ou plusieurs programmes, lesdits un ou plusieurs programmes comprenant des instructions pour, lors de l'exécution desdits un ou plusieurs programmes par un ordinateur comprenant une unité de traitement couplée de manière opérationnelle à des moyens mémoire et à un module d'interface entrées/sorties, conduire l'ordinateur à mettre en œuvre un procédé d'assistance auditive selon l'une quelconque des revendications 1 à 9.

## Patentansprüche

1. Hörhilfeverfahren, das von einer Computervorrichtung (300) durchgeführt wird, das Verfahren umfassend:
- Empfangen (201) von Daten in Bezug auf ein Tonsignal;
- Empfangen (204) von Kontextdaten, die den empfangenen Daten in Bezug auf das Tonsignal zugeordnet sind;
- Bestimmen (203) einer Kategorie des Tonsignals auf der Grundlage der empfangenen Daten und der empfangenen Kontextdaten;
- Bestimmen (202) einer Information, die für eine Position einer Quelle (105) des Tonsignals repräsentativ ist;
- Bestimmen (205) einer Lichtvorgabe in Abhängigkeit von der bestimmten Kategorie des Tonsignals und in Abhängigkeit von der Information, die für die Position der Quelle (105) repräsentativ ist; und
- Übertragen der Lichtvorgabe an mindestens einen Lichtemitter (106, 107).

2. Verfahren nach Anspruch 1, wobei das Bestimmen (203) der Kategorie des Tonsignals ein Auswählen aus einer Menge von vorbestimmten Kategorien in Abhängigkeit von mindestens einem Merkmal des Tonsignals umfasst.

3. Verfahren nach Anspruch 2, wobei das mindestens eine Merkmal des Tonsignals mindestens eins von einer Dauer, einer Frequenz, einer Stärke, einem Energiepegel des Tonsignals, einem Einfallwinkel des Tonsignals oder einer Menge in Bezug auf einen Schalldruck umfasst.

4. Verfahren nach einem der vorhergehenden Ansprüche, wobei das Bestimmen (203) der Kategorie des Tonsignals eine Suche nach einer Schallsignatur in einer Datenbank umfasst.

5. Verfahren nach einem der vorhergehenden Ansprüche, wobei die Kontextdaten Geolokalisierungsdaten umfassen.

6. Verfahren nach einem der vorhergehenden Ansprüche, wobei die Vorgabe mindestens einen Steuerungsparameter des Lichtemitters umfasst, wobei der Parameter mindestens eine von einer Lichtstärke, einer Farbe oder eine Blinkfrequenz umfasst.

7. Verfahren nach einem der vorhergehenden Ansprüche, ferner umfassend, nach der Übertragung der Lichtvorgabe:
- Empfangen (206) von Informationen in Bezug auf eine Bewegung eines Benutzers nach der Emission eines Lichtsignals durch den Lichtemitter;
- Bestimmen (205) einer aktualisierten Lichtvorgabe in Abhängigkeit von den Informationen in Bezug auf die Bewegung des Benutzers; und
- Übertragen der aktualisierten Lichtvorgabe an den Lichtemitter (106, 107).

8. Verfahren nach einem der vorhergehenden Ansprüche, ferner umfassend:
- Erhalten von Informationen in Bezug auf einen Benutzer; und
- Durchführen einer prädiktiven Analyse an den gewonnenen Informationen;
wobei die Lichtvorgabe ferner in Abhängigkeit von der durchgeführten prädiktiven Analyse bestimmt (205) wird.

9. Verfahren nach einem der vorhergehenden Ansprüche, ferner umfassend, nach der Übertragung der Lichtvorgabe:
- Empfangen (206), über eine Mensch-Maschine-Schnittstelle, eines Anweisungsdatenelements von einem Benutzer nach der Emission eines Lichtsignals durch den Lichtemitter;
- Bestimmen (205) einer aktualisierten Vorgabe in Abhängigkeit von dem empfangenen Anweisungsdatenelement; und
- Übertragen der aktualisierten Vorgabe an den Lichtemitter (106, 107).

10. Modul einer Hörhilfe-Computervorrichtung (300), wobei das Modul eine Schaltung umfasst, die zur Durchführung eines Verfahrens nach einem der Ansprüche 1 bis 9 ausgebildet ist.

11. Computervorrichtung (300) umfassend ein Hörhilfemodul nach Anspruch 10.

12. System, umfassend:
- eine Computervorrichtung (300) nach Anspruch 11; und
- eine tragbare Vorrichtung (101), die mit der Computervorrichtung verbunden ist,
wobei die tragbare Vorrichtung mindestens ein Mikrofon (102, 103) und mindestens einen Lichtemitter (106, 107) aufweist, wobei die Daten in Bezug auf das Tonsignal ausgehend von einem Tonsignal erhalten werden, das von dem mindestens einen Mikrofon (102, 103) erfasst wird; wobei die Computervorrichtung (300) dazu ausgebildet ist, die Lichtvorgabe an den mindestens einen Lichtemitter (106, 107) der tragbaren Vorrichtung (101) zu übertragen.

13. Computerprogramm, das in einen Speicher geladen werden kann, der einem Prozessor zugeordnet ist, und das Codeabschnitte zur Durchführung eines Verfahrens nach einem der Ansprüche 1 bis 9 bei der Ausführung des Programms durch den Prozessor umfasst.

14. Träger zur nichtflüchtigen Speicherung eines computerausführbaren Programms, das eine Menge von Daten umfasst, die ein oder mehrere Programme repräsentieren, wobei das eine oder die mehreren Programme Anweisungen umfassen, um bei der Ausführung des einen oder der mehreren Programme durch einen Computer mit einer Verarbeitungseinheit, die in Wirkbeziehung an Speichermittel und an ein Eingangs-/Ausgangs-Schnittstellenmodul gekoppelt ist, den Computer dazu zu veranlassen, ein Hörhilfeverfahren nach einem der Ansprüche 1 bis 9 durchzuführen.

## Claims

1. Hearing assistance method, implemented by a computing device (300), the method comprising:
- receiving (201) data in relation to an audio signal;
- receiving (204) contextual data associated with the received data in relation to the audio signal;
- determining (203) a category of the audio signal on the basis of the received data and of the received contextual data;
- determining (202) information representative of a position of a source (105) of the audio signal;
- determining (205) a brightness setpoint on the basis of the determined category of the audio signal and on the basis of the information representative of the position of the source (105); and
- transmitting the brightness setpoint to at least one light emitter (106, 107).

2. Method according to Claim 1, wherein determining (203) the category of the audio signal comprises a selection, from among a set of predetermined categories, on the basis of at least one feature of the audio signal.

3. Method according to Claim 2, wherein the at least one feature of the audio signal comprises at least one from among a duration, a frequency, an intensity, an energy level of the audio signal, an angle of arrival of the audio signal or a quantity in relation to an acoustic pressure.

4. Method according to one of the preceding claims, wherein determining (203) the category of the audio signal comprises searching for an audio signature in a database.

5. Method according to one of the preceding claims, wherein the contextual data comprise geolocation data.

6. Method according to one of the preceding claims, wherein the setpoint comprises at least one control parameter for the light emitter, the parameter comprising at least one from among a brightness, a colour or a flashing frequency.

7. Method according to one of the preceding claims, furthermore comprising, following the transmission of the brightness setpoint:
- receiving (206) information in relation to a movement of a user following the emission of a light signal by the light emitter;
- determining (205) an updated brightness setpoint on the basis of said information in relation to the movement of the user; and
- transmitting the updated brightness setpoint to the light emitter (106, 107).

8. Method according to one of the preceding claims, furthermore comprising:
- obtaining information in relation to a user; and
- performing predictive analysis on said recovered information;
wherein the brightness setpoint is furthermore determined (205) on the basis of the predictive analysis that is performed.

9. Method according to one of the preceding claims, furthermore comprising, following the transmission of the brightness setpoint:
- receiving (206), via a human/machine interface, an item of instruction data for instructing a user following the emission of a light signal by the light emitter;
- determining (205) an updated setpoint on the basis of said received item of instruction data; and
- transmitting the updated setpoint to the light emitter (106, 107).

10. Module of a computing device (300) for hearing assistance, the module comprising a circuit configured so as to implement a method according to any one of Claims 1 to 9.

11. Computing device (300) comprising a hearing assistance module according to Claim 10.

12. System comprising:
- a computing device (300) according to Claim 11; and
- a portable device (101) connected to the computing device,
wherein the portable device comprises at least one microphone (102, 103) and at least one light emitter (106, 107), the data in relation to the audio signal being obtained from the audio signal acquired by the at least one microphone (102, 103);
wherein the computing device (300) is configured so as to transmit the brightness setpoint to the at least one light emitter (106, 107) of said portable device (101).

13. Computer program able to be loaded into a memory associated with a processor and comprising code portions for implementing a method according to any one of Claims 1 to 9 when said program is executed by the processor.

14. Non-transient storage medium for the non-transient storage of a program able to be executed by a computer, comprising a dataset representing one or more programs, said one or more programs comprising instructions, when said one or more programs are executed by a computer comprising a processing unit operationally coupled to memory means and to an input/output interface module, for prompting the computer to implement a hearing assistance method according to any one of Claims 1 to 9.
